# EUROPEAN PATENT SPECIFICATION

(11) **EP 3 961 218 B1**
(45) Date of publication and mention of the grant of the patent: **13.11.2024**
(21) Application number: 21163037.1
(22) Date of filing: 17.03.2021
(51) Int. Cl.: G01N 33/68

(54) **COMPOSITION AND KIT FOR DETECTING TESTICAN-1 FOR IDENTIFYING EXPOSURE TO PARTICULATE MATTER IN LUNG AND METHOD USING THE SAME**
ZUSAMMENSETZUNG UND KIT ZUM NACHWEIS VON TESTICAN-1 ZUR IDENTIFIZIERUNG DER EXPOSITION MIT FEINSTAUB IN DER LUNGE UND VERFAHREN ZUR VERWENDUNG DAVON
COMPOSITION ET KIT POUR LA DÉTECTION DE TESTICAN-1 POUR IDENTIFIER L'EXPOSITION À DES MATIÈRES PARTICULAIRES DANS LES POUMONS ET SON PROCÉDÉ D'UTILISATION

(30) Priority: 26.08.2020 KR 20200108049
(43) Date of publication of application: 02.03.2022
(73) Proprietor: Korea Institute of Science and Technology, Seoul 02792 (KR)
(72) Inventor: LEE, Ji Eun, 02792 Seoul (KR); CHO, Yoonjin, 02792 Seoul (KR); PARK, Yae Eun, 02792 Seoul (KR); SHIN, Jong Hwan, 02792 Seoul (KR); PARK, Hyun Mee, 02792 Seoul (KR); KIM, So Yeon, 02792 Seoul (KR)
(74) Representative: Longland, Emma Louise

(56) References cited:
- KR-B1- 101 869 509
- YU XIAO-FAN ET AL: "The role of miR-130a-3p and SPOCK1 in tobacco exposed bronchial epithelial BEAS-2B transformed cells: Comparison to A549 and H1299 lung cancer cell lines", JOURNAL OF TOXICOLOGY AND ENVIRONMENTAL HEALTH. PART A, vol. 82, no. 15, 3 August 2019 (2019-08-03), US, pages 862 - 869, XP055835825, ISSN: 1528-7394, DOI: 10.1080/15287394.2019.1664479
- LEE YURI ET AL: "Testican-1, as a novel diagnosis of sepsis", JOURNAL OF CELLULAR BIOCHEMISTRY, vol. 119, no. 5, 25 January 2018 (2018-01-25), pages 4216 - 4223, XP055835761, ISSN: 0730-2312, Retrieved from the Internet <URL:https://onlinelibrary.wiley.com/doi/full-xml/10.1002/jcb.26661> DOI: 10.1002/jcb.26661

## Description

### BACKGROUND

### 1. Field

The present disclosure relates to use of a composition for detecting Testican-1 for identifying exposure to particulate matter in lungs and a method using the same.

### 2. Description of the Related Art

Fine dust, also called particle matter, is a particulate material that floats in the air and is known to be typically generated from exhaust gases of automobiles, combustion of fossil fuels, road dusts, etc. Particle matter is a Group 1 carcinogen that can cause cancer, designated by the International Agency for Research on Cancer (IARC), and fine dust particles having a diameter of less than 10 µm may be induced into the lung and blood, consequently posing a major threat. It is known that the particulate matter increases the incidence of lung cancer by causing coughing or phlegm due to accumulation of dust in the bronchus, and inducing an inflammation reaction due to the dust accumulating in the pulmonary alveolus, thereby generating active oxygen, resulting in necrosis of tissues and degrading lung functions.

Therefore, since the particulate matter causes an inflammatory disease or leads to a potential risk of lung cancer in a serious case, there are constant demands for discovery of a biomarker capable of predicting a change in the lung due to the particulate matter and quickly responding to a change in risk factors.

### PRIOR ART DOCUMENTS

### PATENT DOCUMENTS

(Patent Document 1) KR10-2018-0002324 A
(Patent Document 2) KR10-2019-0127350 A

### SUMMARY

The provided invention is according to the claims.

An aspect of the disclosure, which is not claimed, is to provide a composition for identifying exposure to particulate matter in a lung, comprising an agent for measuring an expression level of a Testican-1 gene or an expression level of a Testican-1 protein or a fragment thereof.

The term "particulate matter" refers to fine dust particulate matter (PM) floating in the air. The particulate matter may include ionic components, such as nitrates (NO³⁻), ammonium (NH⁴⁺), or sulfate (SO₄²⁻), carbon compounds, metal compounds, polycyclic aromatic hydrocarbons (PAHs), nitro-PAHs, or a combination thereof. The particulate matter may be categorized according to its particle size: particulate matter (PM₁₀) with a diameter of not greater than 10 µm and ultrafine particulate matter (PM_{2.5}) with a diameter of not greater than 2.5 µm. PM₁₀ may include PM_{2.5}. The particulate matter may have an average diameter of about 50 µm or less, about 40 µm or less, about 30 µm or less, about 20 µm or less, about 10 µm or less, about 9 µm or less, about 8 µm or less, about 7 µm or less, about 6 µm or less, about 5 µm or less, about 4 µm or less, about 3 µm or less, about 2.5 µm or less, about 2 µm or less, about 1 µm or less, or a combination thereof.

The "lung" may be a lung tissue or a lung cell. The lung cell may be a lung fibroblast.

The "exposure to particulate matter in a lung" may refer to a case in which particulate matter passes through the respiratory organ of a subject to come into contact with lung tissues or lung cells of the subject.

Testican-1 may also be called SPOCK or SPOCK1. Testican-1 may be a protein encoded by SPOCK1 gene in humans. Testican-1 may be proteoglycan including chondroitin sulfate and heparan sulfate. Testican-1 may include an amino acid sequence of Uniprot ID NO: Q08629 in humans. Testican-1 may include an amino acid sequence of Uniprot ID NO: Q62288 in mice.

The fragment may refer to a part of Testican-1 protein, and may be immunogenic polypeptide.

The Testican-1 gene refers to a nucleic acid encoding Testican-1 protein. The Testican-1 gene may be a SPOCK1 gene.

The expression level of Testican-1 gene may be an expression level of mRNA encoding Testican-1 protein. The expression level of mRNA may be a relative or absolute amount of mRNA. The measuring of the expression level of Testican-1 gene may be measuring an amount of mRNA.

The expression level of Testican-1 protein or a fragment thereof may be a relative or absolute amount of Testican-1 protein or the fragment thereof. The measuring of the expression level of the Testican-1 protein or the fragment thereof may be measuring an amount of Testican-1 protein or the fragment thereof.

The agent may be an antibody or an antigen-binding fragment, or aptamer, specifically binding to Testican-1 protein or the fragment thereof. The antibody may be a polyclonal antibody or a monoclonal antibody. The term "antibody" may be used interchangeably with the term "immunoglobulin". The antibody may be a polyclonal antibody or a monoclonal antibody. The antibody may be a full-length antibody. The antigen-binding fragment refers to a polypeptide including an antigen-binding site. The antigen-binding fragment may be a single-domain antibody, Fab, Fab', or scFv. The antibody or the antigen-binding fragment may be adhered to a solid support material. The solid support material may be, for example, a surface of a metal chip, plate, or well. The antibody or the antigen-binding fragment may be an anti-Testican-1 antibody or an antigen-binding fragment. The aptamer may be a single stranded nucleic acid (DNA, RNA or modified nucleic acid) or peptide specifically binding to Testican-1 protein or a fragment thereof.

The agent may be a nucleic acid including a polynucleotide identical to or complementary to a polynucleotide encoding Testican-1 protein or a fragment thereof. The nucleic acid may be a primer, a probe, or an anti-sense oligonucleotide. The primer, the probe, or the anti-sense oligonucleotide may be labeled with a fluorescent substance, a chemiluminescent substance, or a radioactive isotope, at an end or inside thereof.

The composition may be a composition for in vitro measuring an expression level of Testican-1 gene or an expression level of Testican-1 protein or a fragment thereof in a lung tissue or a lung cell.

Described herein is a kit for identifying exposure to particulate matter in a lung, comprising an agent for measuring an expression level of a Testican-1 gene or an expression level of a Testican-1 protein or a fragment thereof.

The Testican-1, Testican-1 gene, fragment, expression level, lung, and exposure to particulate matter, are the same as described above.

The kit may further include a sample necessary for identifying exposure to particulate matter. For immunological detection of the antibody or the antigen-binding fragment thereof, the kit may include a substrate, a suitable buffer solution, a chromogenic enzyme, a secondary antibody labeled with a fluorescent substance, or a chromogenic substrate. For detection of nucleic acids, the kit may include a polymerase, a buffering agent, a nucleic acid, a coenzyme, a fluorescent substance, or a combination thereof. The polymerase may be, for example, a Tag polymerase.

The kit may be a kit for in vitro measuring an expression level of Testican-1 gene or an expression level of Testican-1 protein or a fragment thereof in a lung tissue or a lung cell.

Another aspect is to provide a method for detecting Testican-1 for providing information required in identifying exposure to particulate matter in a lung, the method comprising: measuring an expression level of Testican-1 gene or an expression level of Testican-1 protein or a fragment thereof in a biological sample isolated from a subject with suspected exposure to particulate matter; comparing the measured expression level with that in a normal control group; and identifying whether the Testican-1 expression level has increased as compared with that in the normal control group, wherein an increase in expression level indicates a high probability of exposure to particulate matter in the lung.

The Testican-1, Testican-1 gene, fragment, expression level, lung, and exposure to particulate matter, are the same as described above.

The method comprises measuring an expression level of Testican-1 gene or an expression level of Testican-1 protein or a fragment thereof in a biological sample isolated from a subject with suspected exposure to particulate matter.

The method may be performed in vitro.

The subject may be a mammal, for example, such as a human being, a cow, a horse, a pig, a dog, a sheep, a goat, or a cat. The subject may be a subject suspected to be exposed or have been exposed to particulate matter.

The biological sample refers to a sample obtained from the subject. The biological sample may include a lung tissue, a lung cell, blood, plasma, serum, bone marrow fluid, lymph fluid, saliva, tear fluid, mucosal fluid, amniotic fluid, or a combination thereof. The lung cell may be a lung fibroblast. The biological sample include a protein sample or a nucleic acid obtained from the sample.

The measuring may include incubating the biological sample with an antibody or an antigen-binding fragment thereof, or an aptamer that specifically binds to Testican-1 protein or a fragment thereof. The measuring may be performed by electrophoresis, immunoblotting, enzyme-linked immunosorbent assay (ELISA), protein chip, immunoprecipitation, microarray, electron microscopy, or a combination thereof. The electrophoresis may be SDS-PAGE, isoelectric point electrophoresis, 2D electrophoresis, or a combination thereof.

The measuring may include incubating the biological sample with a polynucleotide identical to or complementary to a polynucleotide encoding Testican-1 protein or a fragment thereof. The measuring may be performed by northern blotting or polymerase chain reaction (PCR). The PCR may be real time PCR or reverse transcription PCR.

The method includes comparing the measured expression level with that in a normal control group. The method includes identifying whether the Testican-1 expression level has increased as compared with that in the normal control group. The normal control group refers to a group without exposure to particulate matter, and is referred to as a negative control group. In the method, the amount of the measured expression level of Testican-1 from sample exposed to particulate matter may be increased compared to the expression level of Testican-1 derived from the sample of the normal control group. For example, when the amount of detected Testican-1 protein is greater than that in the normal control group, the subject may be diagnosed as having a high probability of being exposed or being exposed to particulate matter. The subject identified or diagnosed with exposure to particulate matter may be determined to suffer from or have a high probability of a respiratory disease, a vascular disease, a brain disease, a cancer, or a combination thereof. The respiratory disease may be cold, nasal inflammation, asthma, sinusitis, or pneumonia. The vascular disease may be arteriosclerosis, arrhythmia, angina, myocardial infarction, heart failure, heart attack, or a combination thereof. The brain disease may be stroke, ischemic stroke, cognitive disorder, dementia, depression, or a combination thereof. The cancer may be a lung cancer or a bladder cancer.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a workflow of an experimental method for analyzing a protein biomarker; and
FIG. 2 is a graph showing intensities of Testican-1 depending on concentrations of particulate matter exposed to lung cells.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, the present disclosure will be described in greater detail through examples. However, the following examples are provided for illustrating one or more specific examples, and the present disclosure is not limited thereto.

### Example 1. Identification of differentially expressed proteins depending on ceoncentrations of particulate matter exposed to lung cells

### 1. Culture of lung cells

CCD-8Lu (ATCC) as a lung normal fibroblast cell line of a human lung was culturedin the presence of a medium with 10% (v/v) fetal bovine serum (Gibco^{®}, Thermo Fisher Scientific Inc.) and 1% (w/v) antibiotic-antimycotic (Gibco^{®}, Thermo Fisher Scientific Inc.) added to an Eagle's Minimum Essential Medium (EMEM, BD Bioscience) in a 37°C, 5% CO₂ incubator.

### 2. Treatment of lung cells with particulate matter

A particulate matter powder (SRM2975, National Institute of Standards & Technology) was dissolved in a phosphate buffered saline (PBS) to prepare 8 mg/mL of a particulate matter solution. The prepared particulate matter solution was diluted with an EMEM medium to prepare concentrations of 0 µg/mL, 100 µg/mL, 200 µg/mL, and 400 µg/mL. The prepared particulate matter solutions were added to CCD-8Lu cell line and incubated at 37 °C for 24 hours or 48 hours.

### 3. Preparation of samples for mass spectral analysis

Proteins that are differentially expressed in CCD-8Lu cells treated with particulate matter were analyzed.

Specifically, lysis buffer (50 mM Tris-HCl (pH 7.5), 7 M urea, 2 M thiourea, 1 mM EDTA, 150 mM NaCl, a protease inhibitor cocktail, and phosphatase inhibitor cocktail (Roche) were added to CCD-8Lu cells treated with particulate matter in the same manner as in Example 1.2, and then the CCD-8Lu cells harvested using a scraper. The harvested cells were transferred to a 1.5 mL Lo-bind tube and lysed using ultrasonic processor (Sonics & Materials, Inc.) to extract proteins from the CCD-8Lu cells. The extracted proteins were quantified using a Bradford assay kit.

Urea was added to the extracted proteins so as to have a final urea concentration of 8 M, and 0.5 µmοl of TCEP (tris(2-carboxyethyl)phosphine) was added thereto and incubated for 1 hour at room temperature to reduce disulfide bonds. Afterward, 1 µmοl of IAA (2-lodoacetamide) was added and then incubated at room temperature under a dark condition for about 1 hour for protein alkylation. 25 mM of an ammonium bicarbonate solution was added to the protein reaction product to reduce the concentration of urea in the protein samples to 2 M or less. The proteins were incubated at 30 °C for 2 hours in the presence of 3 mAU of endoproteinase LysC and then incubated at 37 °C overnight in the presence of trypsin. The obtained peptide sample was purified using a SepPak^{®} tC18 cartridge, followed by vacuum drying. The dried peptide sample was suspended in 100 mM of tetraethylammonium bromide (TEAB), and then the peptide was quantified.

### 4. Tandem mass tag (TMT)-labeling of peptide samples

TMT labeling reagents (Thermo Fisher Scientific) were dissolved in 41 µl of acetonitrile and then added to 100 µg of a peptide sample prepared in the same manner as in Example 1.3. As a negative control group, a peptide sample derived from CCD-8Lu cells incubated for 24 hours or 48 hours without treatment with particulate matter was used. After the labeling reaction was made by incubation at 20 °C for 1 hour, 8 µl of 5% (w/v) hydroxylamine was added and incubated at 20 °C for 1 hour to stop the labeling process. The TMT-labeled peptide samples were combined using a single tube, and then purified using a SepPak^{®} tC18 cartridge, followed by vacuum drying.

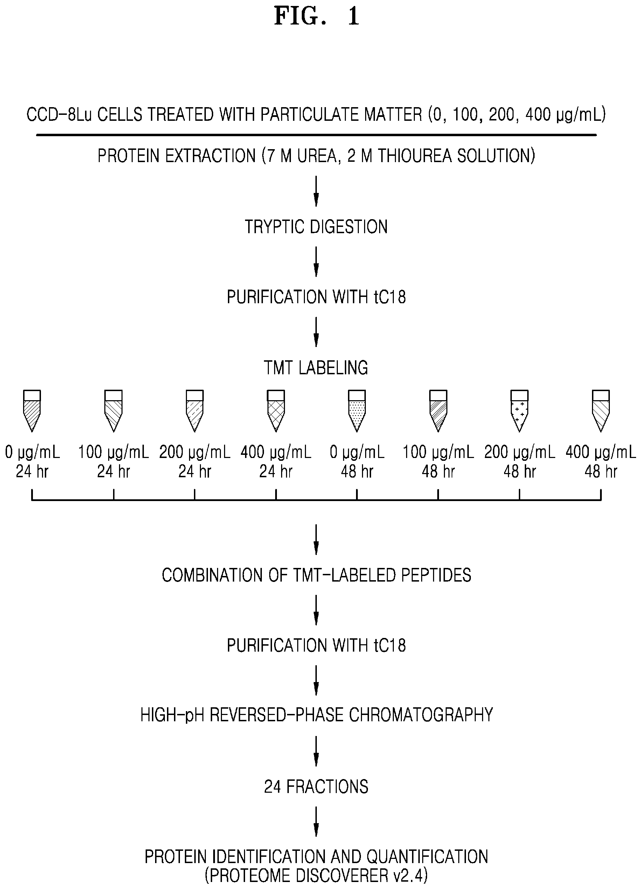

The dried peptide sample was suspended in 10 mM of ammonium formate (pH 10), injected into a C18 column (250 mm × 4.6 mm, 3.5 µm, 130 Å; Waters) and fractionated on an Agilent 1290 ultrahigh performance liquid chromatography (UHPLC) system at a flow rate of 0.5 mL/min.. 96 fractions were combined to give 24 fractions, and the fractions were then dried (FIG. 1).

### 5. Mass spectrometry (MS) analysis of peptide samples

Each 5 µl of the samples obtained in the same manner as Example 1.4 was injected into a reverse-phase PepMap RSLC C18 column (50 cm × 75 µl ) on Ultimate 3000 system. The column was equilibrated with 100% buffer A (100% water containing 0.1% (v/v) formic acid). 80% acetonitrile containing 0.1% (v/v) formic acid was used as a buffer B. The flow rate was 300 nL/min under the following gradient conditions:
0 minute: 93% buffer A and 7% buffer B,
0 minute to 15 minutes: 7% buffer B,
15 minutes to 95 minutes: 7% to 40% buffer B,
95 minutes to 95.1 minutes: 40% to 95% buffer B,
95.1 minutes to 105 minutes: 95% buffer B, and
105 minutes to 105.1 minutes: 7% buffer B.

A nano high-performance liquid chromatography (HPLC) system was connected to Orbitrap Fusion Lumos Tribrid mass spectrometer. Survey full-scan MS spectra (400 to 2,000 m/z) were obtained with 1 microscan and 120,000 resolution, precursors were screened, and a preview mode for measuring a charge state was set. Through preview survey scanning, MS/MS spectra for 20 most intense ions were obtained from full-scanning ion traps with the following options: isolation width of 0.7 m/z; normalized collision energy of 35%; and a dynamic exclusion duration of 50 sec.

The respective LC-MS/MS files were analyzed using the SEQUEST algorithm within Proteome Discoverer 2.4. MS and MS/MS data were searched by comparison with SwissProt human database. The searches were limited such that two missed cleavages were allowed for tryptic peptides. The searches were performed with fixed modification on carboamidomethylation of cysteines (+57.021 Da) and TMT labeling of lysine and peptide N-terminal (+229.163 Da), and variable modification on oxidation of methionine (+15.995 Da). Mass tolerances for MS/MS data and MS data were set to 0.6 Da and 10 ppm, respectively. For decoy database searches, a total of two target values, that is, 0.01 as a strict false discovery rate (FDR) and 0.05 as an eased FDR, were employed.

### 6. Quantitative analysis of labeled peptides

Relative amounts of proteins existing in cells treated with particulate matter in concentrations of 0, 100, 200, or 400 µg/mL for 24 hours or 48 hours were calculated based on the signal to noise values of reporter ions from the labeled peptides. The signal to noise values of reporter ions of the respective peptides were obtained using Proteome Discoverer 2.4. To obtain statistically significant proteins based on the signal to noise values for the respective proteins, Perseus software was used. Even when no protein exists or proteins detected, if any, fall short of a detection limit, for compensating for null values and calculating a difference in the fold change among the respective samples, missing values were complemented by downshifting 0.3 and 1.8 in width from a normal distribution.

With regard to statistical significance, ANOVA test was performed on signal to noise values obtained three technical replicates of LC-MS/MS runs, FDR-adjusted p-values were obtained using a Benjamini-Hochberg correction, and, when the FDR-adjusted p-values were less than 0.05, the protein samples were considered to be statistically significant.

### 7. Identification of proteins differentially expressed in lung cells exposed to particulate matter

From the results of protein identification performed in the same manner as described in Example 1.5, it was confirmed that a total of 7710 proteins were identified in all of the cell samples exposed to particulate matter according to particulate matter concentrations. Out of the identified proteins, 7368 proteins had signal to noise values suitable for quantitative analysis. When ANOVA statistical analyses were performed on the signal-to-noise based abundance values in the 7368 proteins, 2244 proteins having an FDR-adjusted p-value of less than 0.05 were obtained.,Some of the statistically significant proteins are listed in Table 1.

**[Table 1]**

| Un iProt Acce ssion No. | Protein Name | Exposure to particulate matter | | | | | | | | FDR p - val ue |
|---|---|---|---|---|---|---|---|---|---|---|
| | | 24 hr | | | | 48 hr | | | | |
| | | 0 µg/mL | 1000 µg/mL | 2000 µg/mL | 4000 µg/mL | 0 µg/mL | 1000 µg/mL | 2000 µg/mL | 4000 µg/mL | |
| Q8W W Q8 | Stabilin-2 | 7 | 21 | 46 | 109 | 7 | 23 | 83 | 213 | 5.22E-05 |
| Q08629 | Testican-1 | 112 | 116 | 198 | 340 | 103 | 229 | 341 | 826 | 4.03E-07 |
| Q09666 | Neuroblast differentiation-associated protein AHNAK | 11208 9 | 115672 | 122006 | 119705 | 133488 | 144858 | 150985 | 157927 | 1.70E-15 |
| Q15149 | Plectin | 63476 | 64854 | 65196 | 64610 | 66936 | 69208 | 67137 | 63865 | 0.0340 59 |
| P35579 | Myosin-9 | 61945 | 63975 | 65138 | 62931 | 58779 | 66231 | 61989 | 58232 | 1.32E-06 |
| P08670 | Vimentin | 56391 | 62550 | 57955 | 55690 | 61048 | 82766 | 73385 | 74149 | 8.85E-08 |
| P60709 | Actin, cytoplasmic 1 | 39766 | 47691 | 41121 | 44084 | 41142 | 50932 | 49527 | 48686 | 1.79E-08 |
| Q14315 | Filamin-C | 28862 | 29194 | 30716 | 31459 | 29113 | 33707 | 29644 | 30791 | 9.25E-07 |
| Q14204 | Cytoplasmic dynein 1 heavy chain 1 | 26535 | 26590 | 27018 | 27372 | 25166 | 22854 | 27094 | 27062 | 0.0019 77 |
| P21333 | Filamin-A | 25427 | 27758 | 29355 | 30611 | 24413 | 30102 | 25990 | 27106 | 2.48E-07 |
| P04406 | Glyceraldehyd e-3-phosphate dehydrogenas e | 24353 | 26823 | 26113 | 27199 | 24615 | 32183 | 31998 | 34510 | 2.16E-10 |
| Q9Y490 | Talin-1 | 23530 | 24268 | 24043 | 24079 | 23192 | 27793 | 21877 | 21322 | 4.92E-06 |
| P13639 | Elongation factor 2 | 25148 | 24349 | 23505 | 24076 | 21920 | 19094 | 18753 | 17693 | 0.0001 41 |
| P06733 | Alpha-enolase | 22380 | 23647 | 23305 | 23541 | 24024 | 37593 | 29857 | 30035 | 1.51E-06 |
| P26038 | Moesin | 21886 | 20957 | 19682 | 20197 | 18435 | 15620 | 16390 | 15171 | 3.12E-10 |
| P02545 | Prelamin-A/C | 22557 | 24106 | 24575 | 24226 | 23734 | 26410 | 20369 | 21045 | 0.0012 04 |
| P08238 | Heat shock protein HSP 90-beta | 19977 | 19718 | 19906 | 20660 | 19108 | 14906 | 17150 | 15910 | 3.65E-07 |
| P07437 | Tubulin beta chain | 20018 | 21430 | 20363 | 21264 | 22690 | 29412 | 27800 | 28033 | 2.77E-11 |
| P11142 | Heat shock cognate 71 kDa protein | 20948 | 21495 | 21202 | 21118 | 20477 | 31265 | 21539 | 22983 | 1.06E-05 |
| Q13813 | Spectrin alpha chain, non-erythrocytic 1 | 20061 | 21026 | 23270 | 23891 | 21845 | 25026 | 27840 | 30462 | 1.11E-09 |
| P07237 | Protein disulfide-isomerase | 19343 | 18991 | 20769 | 21139 | 19598 | 27933 | 22612 | 21707 | 0.0343 21 |
| P11021 | Endoplasmic reticulum chaperone BiP | 19662 | 19988 | 20707 | 20768 | 19174 | 28544 | 22890 | 23872 | 3.80E-06 |
| P14618 | Pyruvate kinase PKM | 18739 | 19027 | 18155 | 18288 | 18577 | 21206 | 18964 | 17754 | 0.0160 37 |
| P08133 | Annexin A6 | 18733 | 19005 | 19483 | 20361 | 16942 | 17602 | 17409 | 16944 | 0.0023 95 |
| Q9UPN 3 | Microtubule-actin crosslinking factor 1, isoforms 1/2/3/5 | 17511 | 19881 | 20242 | 20472 | 17834 | 19485 | 21080 | 21811 | 8.18E-05 |
| P68104 | Elongation factor 1-alpha 1 | 17811 | 17694 | 17564 | 17569 | 15569 | 19470 | 17087 | 16436 | 0.0002 33 |
| Q9NZM 1 | Myoferlin | 16600 | 17921 | 18506 | 18892 | 16181 | 17466 | 22456 | 21854 | 2.17E-05 |
| O43707 | Alpha-actinin-4 | 16344 | 17511 | 17911 | 18814 | 17547 | 19990 | 18774 | 19342 | 0.0008 84 |
| Q00610 | Clathrin heavy chain 1 | 15554 | 15540 | 15259 | 15567 | 14207 | 13961 | 14063 | 13222 | 0.0162 79 |
| P00558 | Phosphoglyce rate kinase 1 | 15897 | 16711 | 16417 | 15977 | 15957 | 19829 | 16402 | 15887 | 0.0145 54 |
| P46940 | Ras GTPase-activating-like protein IQGAP1 | 16078 | 15871 | 14984 | 10822 | 14991 | 12082 | 14328 | 11213 | 1.79E-08 |
| O75369 | Filamin-B | 15162 | 15289 | 15610 | 15045 | 15465 | 16332 | 14091 | 13993 | 0.1830 9 |
| Q01995 | Transgelin | 15295 | 15372 | 19699 | 18863 | 17674 | 25034 | 22972 | 24202 | 4.38E-09 |
| P08758 | Annexin A5 | 13916 | 14712 | 14581 | 15133 | 14592 | 18021 | 16600 | 16611 | 6.47E-05 |
| Q9BQE 3 | Tubulin alpha-1C chain | 13634 | 14918 | 14460 | 15518 | 14751 | 15754 | 18410 | 17711 | 5.26E-07 |
| P07814 | Bifunctional glutamate/proli ne--tRNA ligase | 13217 | 13117 | 13316 | 13898 | 12395 | 9987 | 10118 | 9523 | 7.96E-11 |
| P27816 | Microtubule-associated protein 4 | 13559 | 13363 | 13870 | 13736 | 13838 | 13134 | 11696 | 12020 | 1.37E-05 |

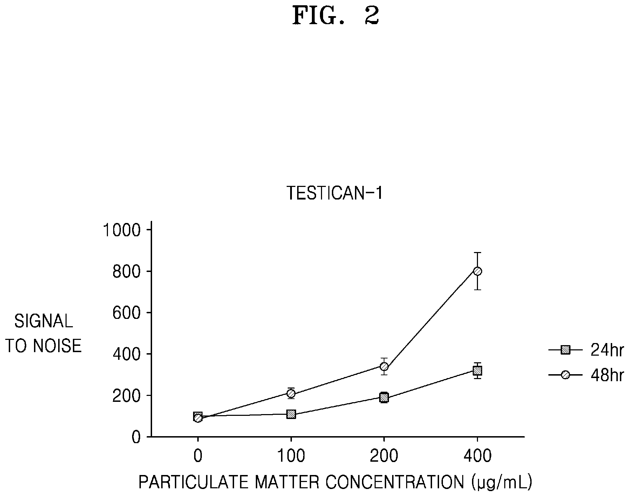

As shown in Table 1, it was confirmed that the expression levels of Testican-1 in statistically significant proteins were increased as the particulate matter concentration increased. A graph showing signal to noise values of Testican-1 according to particulate matter concentrations is shown in FIG. 2.

Accordingly, the Testican-1 was identified as a biomarker for identifying exposure to particulate matter in a lung.

According to an aspect, the composition or kit for detecting Testican-1 for identifying exposure to particulate matter in a lung and the method for detecting Testican-1 using the same may be used in identifying the exposure to particulate matter in a lung in an objective and easy manner with high accuracy and high specificity.

## Claims

1. In vitro use of a composition for identifying exposure to particulate matter in a lung,
wherein the composition comprises an agent for measuring an expression level of Testican-1 gene or an expression level of Testican-1 protein or a fragment thereof; and
wherein identifying exposure to particulate matter in a lung is by identifying an increase in expression level of Testican-1 gene or an increase in expression level of Testican-1 protein or a fragment thereof.

2. The in vitro use of a composition of claim 1, wherein the agent comprises
an antibody or an antigen-binding fragment, or an aptamer, which specifically binds to Testican-1 protein or a fragment thereof; or
a nucleic acid comprising a polynucleotide identical to or complementary to a polynucleotide encoding Testican-1 protein or a fragment thereof.

3. The in vitro use of a composition of claim 2, wherein the antibody is a polyclonal antibody or a monoclonal antibody.

4. The in vitro use of a composition of claim 2, wherein the nucleic acid is a primer, a probe, or an anti-sense oligonucleotide.

5. A method for detecting Testican-1 for providing information required in identifying exposure to particulate matter in a lung, the method comprising:
measuring an expression level of Testican-1 gene or an expression level of Testican-1 protein or a fragment thereof in a biological sample isolated from a subject with suspected exposure to particulate matter;
comparing the measured expression level with that in a normal control group; and
identifying whether the Testican-1 expression level has increased as compared with that in the normal control group, and
wherein an increase in expression level indicates a high probability of exposure to particulate matter in the lung.

6. The method of claim 5, wherein the biological sample is a lung tissue, a lung cell, blood, plasma, serum, bone marrow fluid, lymph fluid, saliva, tear fluid, mucosal fluid, amniotic fluid, or a combination thereof.

7. The method of claim 5, wherein the measuring comprises incubating the biological sample with an antibody or an antigen-binding fragment thereof, or an aptamer, which specifically binds to Testican-1 protein or a fragment thereof.

8. The method of claim 5, wherein the measuring comprises incubating the biological sample with a polynucleotide identical to or complementary to a polynucleotide encoding Testican-1 protein or a fragment thereof.

9. The method of claim 5, wherein the measuring is performed by electrophoresis, immunoblotting, enzyme-linked immunosorbent assay (ELISA), protein chip, immunoprecipitation, microarray, electron microscopy, or a combination thereof.

## Patentansprüche

1. In-vitro-Verwendung einer Zusammensetzung zum Bestimmen der Belastung einer Lunge mit Feinstaub, wobei die Zusammensetzung ein Mittel zum Messen einer Expressionsrate des Testican-1-Gens oder einer Expressionsrate des Testican-1-Proteins oder eines Fragments davon umfasst, und
wobei das Bestimmen der Belastung einer Lunge mit Feinstaub durch Bestimmen eines Anstiegs der Expressionsrate des Testican-1-Gens oder eines Anstiegs der Expressionsrate des Testican-1-Proteins oder eines Fragments davon erfolgt.

2. In-vitro-Verwendung einer Zusammensetzung nach Anspruch 1, wobei das Mittel umfasst:
einen Antikörper oder ein Antigen-bindendes Fragment oder ein Aptamer, der/das spezifisch an das Testican-1-Protein oder ein Fragment davon bindet; oder
eine Nukleinsäure, die ein Polynukleotid umfasst, das mit einem Polynukleotid identisch oder komplementär ist, das das Testican-1-Protein oder ein Fragment davon codiert.

3. In-vitro-Verwendung einer Zusammensetzung nach Anspruch 2, wobei der Antikörper ein polyklonaler Antikörper oder ein monoklonaler Antikörper ist.

4. In-vitro-Verwendung einer Zusammensetzung nach Anspruch 2, wobei die Nukleinsäure ein Primer, eine Sonde oder ein Antisense-Oligonukleotid ist.

5. Verfahren zum Nachweisen von Testican-1 zum Verfügbarmachen von Informationen, die zum Bestimmen der Belastung einer Lunge mit Feinstaub erforderlich sind, wobei das Verfahren umfasst:
Messen einer Expressionsrate des Testican-1-Gens oder einer Expressionsrate des Testican-1-Proteins oder eines Fragments davon in einer biologischen Probe, die aus einem Subjekt isoliert wurde, bei dem der Verdacht auf Feinstaubbelastung besteht;
Vergleichen der gemessenen Expressionsrate mit der einer normalen Kontrollgruppe; und
Bestimmen, ob die Expressionsrate von Testican-1 im Vergleich zu der in der normalen Kontrollgruppe angestiegen ist, und
wobei ein Anstieg der Expressionsrate auf eine hohe Wahrscheinlichkeit einer Belastung der Lunge mit Feinstaub hinweist.

6. Verfahren nach Anspruch 5, wobei die biologische Probe ein Lungengewebe, eine Lungenzelle, Blut, Plasma, Serum, Knochenmarkflüssigkeit, Lymphflüssigkeit, Speichel, Tränenflüssigkeit, Schleimhautflüssigkeit, Fruchtwasser oder eine Kombination davon ist.

7. Verfahren nach Anspruch 5, wobei das Messen das Inkubieren der biologischen Probe mit einem Antikörper oder einem Antigen-bindenden Fragment davon oder einem Aptamer umfasst, der/das spezifisch an das Testican-1-Protein oder ein Fragment davon bindet.

8. Verfahren nach Anspruch 5, wobei das Messen das Inkubieren der biologischen Probe mit einem Polynukleotid umfasst, das mit einem Polynukleotid identisch oder komplementär ist, das das Testican-1-Protein oder ein Fragment davon codiert.

9. Verfahren nach Anspruch 5, wobei das Messen mittels Elektrophorese, Immunblotten, Enzymimmunoassay (ELISA), Proteinchip, Immunpräzipitation, Microarray, Elektronenmikroskopie oder einer Kombination davon durchgeführt wird.

## Revendications

1. Utilisation in vitro d'une composition permettant l'identification d'une exposition à une matière particulaire dans un poumon,
ladite composition comprenant un agent destiné à mesurer le niveau d'expression d'un gène de testicane-1 ou le niveau d'expression d'une protéine de testicane-1 ou d'un fragment de celle-ci ; et
ladite identification d'une exposition à une matière particulaire dans un poumon se faisant par l'identification d'une augmentation du niveau d'expression d'un gène de testicane-1 ou du niveau d'expression d'une protéine de testicane-1 ou d'un fragment de celle-ci.

2. Utilisation in vitro d'une composition selon la revendication 1, ledit agent comprenant
un anticorps ou un fragment de liaison à un antigène, ou un aptamère, qui se lie spécifiquement à une protéine de testicane-1 ou à un fragment de celle-ci ; ou
un acide nucléique comprenant un polynucléotide identique ou complémentaire à un polynucléotide codant une protéine de testicane-1 ou un fragment de celle-ci.

3. Utilisation in vitro d'une composition selon la revendication 2, ledit anticorps étant un anticorps polyclonal ou un anticorps monoclonal.

4. Utilisation in vitro d'une composition selon la revendication 2, ledit acide nucléique étant une amorce, une sonde ou un oligonucléotide antisens.

5. Méthode de détection du testicane-1 permettant de fournir les informations requises dans l'identification d'une exposition à une matière particulaire dans un poumon, la méthode comprenant :
la mesure du niveau d'expression d'un gène de testicane-1 ou du niveau d'expression d'une protéine de testicane-1 ou d'un fragment de celle-ci dans un échantillon biologique prélevé chez un sujet chez lequel une exposition à une matière particulaire est suspectée ;
la comparaison du niveau d'expression mesuré à celui d'un groupe témoin normal ; et
l'identification pour déterminer si le niveau d'expression du testicane-1 a augmenté par rapport à celui dans le groupe témoin normal, et
l'augmentation du niveau d'expression indiquant une probabilité élevée d'exposition à une matière particulaire dans le poumon.

6. Méthode selon la revendication 5, ledit échantillon biologique étant un tissu pulmonaire, une cellule pulmonaire, du sang, du plasma, du sérum, du liquide de moelle osseuse, du liquide lymphatique, de la salive, du liquide lacrymal, du mucus, du liquide amniotique ou une combinaison de ceux-ci.

7. Méthode selon la revendication 5, ladite mesure comprenant l'incubation de l'échantillon biologique avec un anticorps ou un fragment de liaison à un antigène de celui-ci, ou un aptamère, qui se lie spécifiquement à une protéine de testicane-1 ou à un fragment de celle-ci.

8. Méthode selon la revendication 5, ladite mesure comprenant l'incubation de l'échantillon biologique avec un polynucléotide identique ou complémentaire à un polynucléotide codant une protéine de testicane-1 ou un fragment de celle-ci.

9. Méthode selon la revendication 5, ladite mesure étant effectuée par électrophorèse, immunotransfert, dosage par immunosorbant lié à une enzyme (ELISA), puce à protéines, immunoprécipitation, microréseau, microscopie électronique, ou une combinaison de ceux-ci.
